# EUROPEAN PATENT APPLICATION

(11) **EP 1 489 164 A1**
(43) Date of publication of application: **22.12.2004**
(21) Application number: 03013826.7
(22) Date of filing: 18.06.2003
(51) Int. Cl.: C12N 7/04, A61K 35/76, C12N 15/86, A61K 48/00

(54) **Recombinant vaccinia viruses with modified F3 genes, uses thereof**

(71) Applicant: Genelux GmbH, 97074 Würzburg (DE)
(72) Inventor: The designation of the inventor has not yet been filed
(74) Representative: Schüssler, Andrea, Dr.

(57) **Abstract**

Described are genetically modified vaccinia viruses and methods of making and using said vaccinia viruses. In particular, a recombinant vaccinia virus is described wherein (a) the F3 gene, which is assumed to have a nonessential role for virus replication, and (b) the TK gene and/or the HA gene have been inactivated.

## Description

The present invention relates to genetically modified vaccinia viruses (vv) and to methods of making and using said vaccinia viruses. In particular, the present invention relates to a recombinant vaccinia virus wherein (a) the F3 gene, which is assumed to have a nonessential role for virus replication, and (b) the TK gene and/or the HA gene have been inactivated.

Genetically engineered viruses have been frequently used for cancer gene therapy and vaccine therapy. However, limitations on targeted delivery of the viral vehicles to desired tissues result in inefficient therapeutic effects as well as safety concerns. The risks associated with viral vehicles for therapeutic use are several folds. First, systemically administered viral particles may infect organs other than targeted ones. Second, systemically delivered viruses may be highly immunogenic in the host. Third, although certain viruses are attenuated with large deletions, given enough observation time, pathogenesis may still occur in the host. Fourth, since most of the cancer patients are in immunocompromised state, severe viremia may develop when replication-competent viral vehicles are used. Fifth, certain types of viruses, such as retrovirus, can integrate their genome into the host genome. Therefore, it is essential to address safety concerns before live-virus-based therapies can be routinely applied clinically.

Virus-mediated cancer gene therapy is based on the concept that therapeutic proteins expressed from the exogenous genes carried by the virus trigger cytotoxicity specifically towards the tumor cells, while sparing the cells of normal tissues. To achieve such tumor-specific toxicity, there are several requirements of the viral vehicles and therapeutic gene expression cassettes, which can be summarized as "viral vehicle targeting" and "transcription targeting".

In "viral vehicle targeting", ideally the viruses only infect and replicate in tumor tissues. This is proven to be challenging. Researchers have attempted to genetically modify viruses in order to achieve tumor-cell-specific targeting by the viruses (Targeting mechanism: Antibody(on virus)-cell surface protein (on target cells) interaction; protein ligand (on virus)-surface receptor (on target cells) interaction; protease-substrate interaction, in addition to protein ligand (on virus)-surface receptor (on target cells) interaction; conditionally replicating viruses). One of the most frequently used approaches is to modify the composition of virus envelope proteins for tumor-specific tropism. For example, single chain antibodies were inserted as envelope proteins on viruses to target cells expressing corresponding protein ligands on their surface. In a similar effort, viruses are modified to express protein ligands as envelope proteins on the surface. These ligands are complementary to their receptors on the surface of targeted cells. Epidermal growth factor (EGF)/epidermal growth factor receptor (EGFR), Arg-Gly-Asp (RGD) peptide/integrin, are some examples of such ligand/receptor pairs being explored. Although some preliminary experiments using ligand-labeled viruses demonstrated receptor-expression cell targeting, other experiments had mixed results. One explanation is that, after receptor binding and virus internalization, the receptor-virus complexes are rapidly delivered to lysosomes for degradation. To overcome this problem, the endosome translocation domain from Exotoxin A of *Pseudomonas aeruginosa* was inserted into the ligand protein on the virus. Limited success on target cell transfection was observed. In addition to modify viral envelope protein for tumor tropism, another promising approach to achieve "viral vehicle targeting" is to use conditionally replicating virus mutants. For example, mutation in the *E1B* region of the adenovirus genome results in permissive viral replication only in tumor cells that are defective within the p53 pathway. In another example, the adenovirus E1A expression was linked to E2F transcription factors promoter. Therefore, only the tumor cells overexpressing E2F can sustain viral replication. No viral replication occurs in normal nonproliferating tissue with no E2F-1 expression. In addition to adenovirus, conditionally replicating virus mutants are being actively examined in other viruses, such as in herpes simplex virus, reovirus, Newcastle virus, and vaccinia virus.

In "transcription targeting", the therapeutic protein expression needs to specifically occur in tumor tissues. Researchers have used tissue/organ-specific promoters for target gene expression to overcome the fact that viral deliver may occur in tissues other tumors. In addition to using specific promoters, the addition of enhancer repeats further promotes high level of cell-lineage-specific transcriptional activation in tumors. Nevertheless, since most of these promoters are tissue/organ-specific or cell-type-specific, and are not strictly tumor-specific, significant side effects to the tissue/organ of the same lineage to tumors are expected. An alternative approach in "transcription targeting" is using up-regulated promoters that are specific in tumor cells. One such example is the E2F-1 promoter, which has been shown to be derepressed in many tumors due to mutations to upstream regulators. Subsequently, it was shown that linking therapeutic genes to the E2F-1 Promoter allows virus-mediated gene expression selectively in tumors, which resulted in tumor regression.

In addition to cancer gene therapy, live attenuated viruses have also been used for vaccination, such as in cancer vaccination or antitumor immunity. Successful immunization against tumor requires prolonged and high level tumor-specific T-cell-mediated response through virus-delivered antigens or cytokines. To do so, the viral deliver vehicle needs to be specifically targeted to the tumor tissues, and with minimal infection to any other key organs.

One of the viruses that had been used for the above discussed therapeutic applications is vaccinia virus, a member of the poxvirus family. The linear dsDNA viral genome is approximately 200 kb in size, encoding for a total of approximately 200 potential genes. The viral gene expressions can be divided into three stages. In the early stage, gene expressions are mainly for viral replication, and for defense against host immune system. In the late stage, active transcriptions are mainly for viral structural components for building mature viruses. Vaccinia virus strains used so far comprise Ankara (ANK), Copenhagen (COP), Dairen I, IHD, LC16M8, LC16MO, LIPV, Lister (Elstree), LIVP, Tian Tan (TAN), Western Reserve (WR), WR 65-16, Wuyeth etc.

There are two general approaches to develop safe vaccinia virus strains for clinical use. The conventional method to attenuate vaccinia virus is through hundreds of serial passages through chicken embryo fibroblast cultures. The highly attenuated MVA strain is the result of more than 500 passages through cultures in chicken embryo fibroblasts. Through these passages, numerous natural mutations are introduced into the nonessential regions of the genome of the original Ankara strain, particularly in genes of host interactive proteins. The second method, the more modern approach, to attenuate vaccinia virus is by genetic engineering to specifically delete or modify nonessential genes. Since the complete genome sequences and genes involved in pathogenicity in some of the vaccinia virus strains are known, it is possible to selectively mutate nonessential genes in searching for safe mutant strains for clinical use. For example, mutations in thymidine kinase gene (TK), ribonucleotide reductase gene, or hemagglutinin gene (HA) resulted in significantly reduced virulence of the Wyeth vaccine strain. In another example, the highly attenuated NYVAC (vP866) vaccinia strain is a derivative of Copenhagen strain after precise deletion of 18 open reading frames from the viral genome.

Vaccinia virus possesses many attractive features for use in cancer gene therapy and vaccination. It has a broad host and cell type range. Vaccinia is a cytoplasmic virus. It does not insert its genome into the host genome during its life cycle. The vaccinia virus genome has a large carrying capacity for foreign genes. Up to 25 kb of exogenous DNA fragments, which equals to approximately 12% of the genome, can be inserted. Unlike many other viruses which require the host transcription machinery, vaccinia virus can support its own gene expression in the host cell cytoplasm using enzymes encoded in the viral genome. The genomes of several of the vaccinia strains have been completely sequenced. Many of the essential and nonessential genes are identified. Due to high sequence homology among different strains, genomic information from one vaccinia strain can be used for designing and generating recombinant viruses in other strains. And most importantly, the techniques for production of recombinant vaccinia strains by genetic engineering have been well documented (Moss, Curr. Opin. Genet. Dev. 3 (1993), 86-90; Broder and Earl, Mol. Biotechnol. 13 (1999), 223-245; Timiryasova et al., Biotechniques 31 (2001), 534-540).

Many loci in the vaccinia genome have been used as the integration sites of foreign genes. For example, the non-essential TK gene is frequently used as the insertion site. Other frequently used non-essential regions include the HA gene. It has been shown that, even though the same expression cassettes are used, significant variation of protein expression can occur dependent on the location of insertion sites (Coupar et al., J. Gen. Virol. 81 (2000), 431-439).

The non-essential regions of the vaccinia genome, e.g. the tk gene, were originally intended for insertion of foreign genes without significantly affecting viral replication and infection. However, it was shown that tk⁻ vaccinia virus was less virulent than tk⁺ virus. Incidentally, when vaccinia virus carrying mutations in TK gene was injected into mice with tumors, significantly higher viral titers were observed in the tumors than in other tissues, when compared to tk⁺ vaccinia virus. Coupar et al. (J. Gen. Virol. 81 (2000), 431-439) have inserted firefly luciferase gene under the control of vaccinia virus 7.5 kDa promoter into the tk gene or into the *HindIII* F fragment of the viral genome. It was noted that significant higher level of luciferase expression was obtained when the same expression cassette was inserted in the *HindIII* F fragment than when it was inserted in the TK gene. When the genes of IL-2 and IL-4 cytokines under the control of 7.5 kDa promoter were expressed from the same virus, the cytokines expressed from the *HindIII* F insertion site more significantly rescued mice from lethal viral infections than the same cytokines expressed from the tk locus. It was also noted that the sites of insertion also affect tissue distribution of mutant vaccinia virus after intravenous infection. *HindIII F* insertion site significantly promotes luciferase gene expression in the lung tissue as compared to the tk insertion site, even though no differences on viral titer and gene expression levels were seen in spleens and livers. Therefore sites of insertion/mutation may be crucial in designing safe and efficient vaccinia virus vehicles for gene therapy and vaccination.

Historically, vaccinia virus was used to immunize against smallpox infection. More recently, recombinant vaccinia viruses are being developed as vaccines to combat a large variety of diseases. Potent attenuated vaccinia virus triggers cell-mediated immune response. Strategies such as prime/boost vaccination, vaccination with nonreplicating vaccinia virus or the combination of these strategies all showed promising results for the development of safe and effective vaccination protocols. However, unfortunately, the mutant vaccinia viruses used so far to combat diseases like cancer exhibit a variety of shortcomings, one of them is the lack of efficient delivery of the viral vehicle to the desired tissue only, e.g. to the tumor, and/or the other is lack of safety because of the possibilities of serious (but infrequent) complications, e.g., in young children, eczema vaccinatum and encephalitis, in adults disseminated or progressive vaccinia may occur if there is a severe immunodeficiency.

Therefore, it is the object of the present invention to provide a modified vaccinia virus which does not exhibit the disadvantages of the vaccinia viruses of the prior art.

According to the invention this is achieved by the subject matters defined in the claims.

The present invention is based on the applicant's findings that a vaccinia virus having an insertion of a foreign DNA at the NotI site of the F3 gene (strain LIVP), preferably combined with modifications (e.g., inactivation) of the TK gene and/or HA gene, exhibits several of the desired advantages. The goal of the experiments leading to the present invention was to design a recombinant attenuated vaccinia virus with specific genetic mutations for tumor-specific colonization and therefore tumor-specific therapeutic gene delivery. A Genbank data analysis with BLAST (Basic Local Alignment Search Tool) on DNA sequences of different strains of vaccinia virus was performed. Based on this analysis, it was found that in vaccinia virus strain Copenhagen (Goebel et al., Virology 179 (1990), 247-266) the NotI restriction site is located between two open reading frames (ORF) encoding F14L and F15L genes. Therefore, insertion of foreign genes into NotI site of the VV genome strain Copenhagen will not interrupt any vital genes. However, NotI restriction site in VV strain LIVP is located in the ORF encoding F3 gene with unknown functions (Mikryukov et al., Biotekhnologiya 4 (1988), 442-449). Based on this data, it can be concluded that by the insertion of foreign genes into the NotI site the viral F3 gene had been interrupted. In the absence of any published experimental data regarding the expression or role of the VV strain LIVP F3 gene, the ability to delete the F3 gene suggests that it has a nonessential role for virus replication. Although the F3 gene is nonessential for virus replication, the results of the animal experiments suggest that interruption of the F3 gene is correlated with decreased viral virulence and inability to replicate in brain tissue but preferentially to replicate in tumor tissue.

Comparative protein sequence analysis revealed some insights into protein function. It was found that the closest match with the protein encoded by F3 gene (strain LIVP) is a protein named "Prolyl 4-hydroxylase alpha subunit precursor (4-PH alpha)" from the nematode *Caenorhabditis elegans* (Veijola et al., J. Biol. Chem. 269 (1994), 26746-26753). This alpha subunit forms an active alpha beta dimmer with the human protein disulfide isomerase/beta subunit. Prolyl 4-hydroxylase (EC 1.14.11.2) catalyzes the formation of 4-hydroxyprolyne in collagens. The vertebrate enzyme is an alpha 2-beta 2-tetramer, the beta subunit of which is identical to the protein disulfide-isomerase (PDI). The importance of this protein for vaccinia viral replication is unknown. However, it can be expected that deficiency in this protein retargets vaccinia virus into tumor tissue.

The advantages of the modified vaccinia virus of the present application are as follows.

### (a) Toxicity of vaccinia virus

Injection of wild type vaccinia virus VGL (strain LIVP) at 1x10⁷ PFU/mouse (i.v.) causes toxicity in nude mice: three mice out of seven lost the weight and died (one mouse died in one week after virus injection, one mouse died in ten days after virus injection.) However, recombinant vaccinia virus RVGL8 did not show toxic effects in nude mice after i.v. injection at a dose of 1x10⁷ PFU/mouse. There were no readily detectable signs of RVGL8 virus-related toxicity. All mice in this experimental group were doing fine. Therefore, insertion of lacZ gene into NotI site (located in F3 gene) of vaccinia virus genome strain LIVP causes inhibition of the vaccinia virus virulence.

### (b) Vaccinia virus recovery

Very interesting data were received after recovering vaccinia viruses from tumor samples and different organs. VGL virus was recovered from tumor, testes, bladder, and liver and as well as from brain. However, recombinant virus RVGL8 was found mostly in tumors (in mouse #24 virus was found in testes, bladder and liver; in mouse #22 in testes) and no virus was recovered from brain tissue in six tested animals. Therefore, this finding demonstrates the safety of the recombinant vaccinia virus of the invention for gene therapy purposes.

### (c) Tumor-targeting

In summary, vaccinia virus strain LIVP has high transduction efficiency in tumor cells with high levels of gene expression. The results of the experiments leading to the present invention suggest a selective in e.g. vivo replication of F3-interrupted recombinant vaccinia virus (strain LIVP) in tumor cells. Therefore, recombinant vaccinia viruses (e.g. strain LIVP) with the interrupted F3 gene are a useful vector for tumor-directed gene therapy and for detection of tumors and metastases.

Accordingly, the present invention relates to a recombinant vaccinia virus wherein (a) the F3 gene and (b) the TK gene and/or HA gene have been modified.

The term "modified" as used herein relates to a gene preferably encoding a truncated gene product with loss of the gene function.

The term "F3 gene" as used herein relates to the F3 gene of vaccinia virus strain LIVP and, in addition, to a corresponding gene of any vaccinia virus strain encoding a gene product having substantially the same or at least a related biological function.

In a preferred embodiment, the F3 gene, TK gene and/or HA gene have been inactivated, for example by insertion, deletion and/or replacement of nucleotide(s) within the coding region, inactivation of regulatory sequences etc.

For generating modified versions of the F3 gene, TK gene and/or HA gene, respectively, it is possible to use general methods known in the art starting from the published nucleotide sequences of vaccinia virus strains, see, e.g., Mikryukov et al., Biotekhnologiya 4 (1988), 442-449; Goebel et al., Virology 179 (1990), 247-266; Antoine et al., Virology 244 (1998), 365-396; Mayr et al., Zentbl. Bakteriol. Hyg. Abt. 1 Orig. B 167 (1978), 375-390; Ando and Matumoto, Jpn. J. Microbiol. 14 (1979), 181-186; Sugimoto et al., Microbiol. Immunol. 29 (1985), 421-428; Takahashi-Nishimaki et al., J. Gen. Virol. 68 (1987), 2705-2710). These methods include e.g. in vitro recombination techniques, synthetic methods and in vivo recombination methods as described, e.g., in Sambrook et al., Molecular Cloning: A Laboratory Manual, 2nd edition, Cold Spring Harbor Laboratory Press, cold Spring Harbor NY (1989), and in Example 1. The person skilled in the art can isolate the gene encoding the gene product of F3 (or a related gene product) etc. from any vaccinia strain using, e.g., the nucleotide sequence of the F3 gene etc. from VV strain LIVP or a fragment thereof as a probe for screening a library. The person skilled in the art can easily assay whether a modified version of the F3 gene, TK gene and/or HA gene has the desired characteristics by assaying, for example, (a) the reduced or eliminated capability of a recombinant vaccinia virus containing such a mutated form of the gene to replicate in brain tissue and/or (b) the decreased viral virulence, based on the techniques described in the Examples.

In a more preferred embodiment of the recombinant vaccinia virus of the invention, at least the F3 gene has been inactivated by insertion of a foreign DNA sequence. Preferably, the F3 gene and the TK gene have been inactivated by insertion of a foreign DNA sequence.

In an even more preferred embodiment of the recombinant vaccinia virus of the invention, the F3 gene has been modified at the unique single NotI restriction site located within the F3 gene at position 35 or at position 1475 inside of the HindIII-F fragment of vaccinia virus DNA strain LIVP (Mikryukov et al., Biotekhnologiy 4 (1988), 442-449), for example by insertion of a foreign DNA sequence into the NotI digested virus DNA.

Any vaccinia strain is suitable as starting material for constructing the recombinant vaccinia virus of the invention using, e.g., the method of construction described in the Examples with the wild type strain LIVP being preferred.

In a further preferred embodiment, the foreign DNA sequence encodes a protein to be expressed under the control of a regulatory sequence. The present invention has utility in expressing genes in vivo and in vitro. Examples of proteins particularly useful for the present invention are reporter proteins (E. coli ß-galactosidase, ß-glucuronidase, xanthineguanine phosphoribosyltransferase), proteins facilitating detection, i.e., a detectable protein or a protein capable of inducing a detectable signal, (e.g., luciferase, green and red fluorescent proteins, transferrin receptor), proteins useful for tumor therapy (pseudomonas A endotoxin, diphtheria toxin, p53, Arf, Bax, tumor necrosis factor-alfa, HSV TK, E. coli purine nucleoside phosphorylase, angiostatin, endostatin, different cytokines) and many other proteins.

Examples of genes expressible by the vaccinia virus of the present invention include human genes. An exemplary list of genes includes the list of human genes and genetic disorders authored and edited by Dr. Victor A. McKusick and his colleagues at Johns Hopkins University and elsewhere, and developed for the World Wide Web by NCBI, the National Center for Biotechnology Information. Online Mendelian Inheritance in Man, OMIM (TM). Center for Medical Genetics, Johns Hopkins University (Baltimore, Md.) and National Center for Biotechnology Information, National Library of Medicine (Bethesda, Md.), 1999. <<http://www.ncbi.nlm.nih.gov/omim/>>.

Suitable regulatory sequences which, preferably, are functional in a mammalian host cell are well known to the person skilled in the art. Preferably, said regulatory sequence comprises all natural or synthetic vaccinia virus promoters. It is advisable to use poxvirus promoters since cellular and other viral promoters are not recognized by the vaccinia transcriptional apparatus. Strong late promoters are preferable to achieve high levels of expression of the foreign genes. Early and intermediate-stage promoters, however, can also be used. The most versatile and widely used promoters contain early and late promoter elements, e.g. the vaccinia virus early/late promoter p7.5, vaccinia late promoter p11, a synthetic early/late vaccinia pE/L promoter (Patel et al., (1988), Proc. Natl. Acad. Sci. USA 85, 9431-9435; Davison and Moss, (1989), J Mol Biol 210, 749-769; Davison et al., (1990), Nucleic Acids Res. 18, 4285-4286; Chakrabarti et al., (1997), BioTechniques 23, 1094-1097)etc.

Furthermore, the present invention relates to host cells which contain a recombinant vaccinia virus of the invention. These host cells include many mammalian, avian and insect cells and tissues which are susceptible for vaccinia virus infection, including chicken embryo, rabbit, hamster and monkey kidney cells, e.g. CV-1, BSC40, Vero, BSC40 and BS-C-1, and human HeLa cells. Methods of transforming these host cells, of phenotypically selecting transformants etc. are known in the art.

The present invention also relates to a pharmaceutical composition containing a recombinant vaccinia virus of the invention and a suitable pharmaceutical carrier.

Examples of suitable pharmaceutical carriers etc. are well known in the art and include phosphate buffered saline solutions, water, emulsions, such as oil/water emulsions, various types of wetting agents, sterile solutions etc. Such carriers can be formulated by conventional methods and can be administered to the subject at a suitable dose. Administration of the suitable compositions may be effected by different ways, e.g. by intravenous, intraperitoneal, subcutaneous, intramuscular, topical, oral, intranasal or intradermal administration. The route of administration, of course, depends on the nature of the disease, e.g., the kind of tumor, and the kind of virus contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind and stage of the disease, e.g., tumor, general health and other drugs being administered concurrently.

Direct application to the target site can be performed, e.g., by ballistic delivery, as a colloidal dispersion system or by catheter to a site in artery. The colloidal dispersion systems which can be used for delivery of the recombinant vaccinia viruses of the invention include macromolecule complexes, nanocapsules, microspheres, beads and lipid-based systems including oil-in-water emulsions (mixed), micelles, liposomes and lipoplexes. The preferred colloidal system is a liposome. Organ-specific or cell-specific liposomes can be used in order to achieve delivery only to the desired tissue. The targeting of liposomes can be carried out by the person skilled in the art by applying commonly known methods. This targeting includes passive targeting (utilizing the natural tendency of the liposomes to distribute to cells of the RES in organs which contain sinusoidal capillaries) or active targeting (for example by coupling the liposome to a specific ligand, e.g., an antibody, a receptor, sugar, glycolipid, protein etc., by well known methods). In the present invention monoclonal antibodies are preferably used to target liposomes to specific tissues, e.g. tumor tissue, via specific cell-surface ligands.

In order to achieve expression only in the target organ, e.g., a tumor to be treated, the foreign DNA sequence can be linked to a tissue specific promoter and used for gene therapy. Such promoters are well known to those skilled in the art (see e.g. Zimmermann et al., (1994) Neuron 12, 11-24; Vidal et al.; (1990) EMBO J. 9, 833-840; Mayford et al., (1995), Cell 81, 891-904; Pinkert et al., (1987) Genes & Dev. 1, 268-76).

The present invention also relates to the use of a recombinant vaccinia virus described above or a recombinant vaccinia virus having a modification (with the kind of modification corresponding to the modifications as defined for the vaccinia virus containing a modification of the F3 gene and the TK gene and/or the HA gene) of the F3 gene, the TK gene or the HA gene only for the preparation of a pharmaceutical composition for gene therapy, preferably cancer gene therapy, or vaccine therapy. Such a vaccine can be used to stimulate humoral and/or cellular immune response, induce strong cytotoxic T lymphocytes responses in subjects who may benefit from such responses, e.g., by providing prophylactic and therapeutic effects against tumors and other infectious diseases, by rejection of cells from tumors or lesions using vaccinia viruses which express viral antigens (Earl et al. (1986), Science 234, 728-831; Lathe et al. (1987), Nature (London) 326, 878-880), cellular tumor-associated antigens (Bernards et al.,(1987), Proc. Natl. Acad. Sci. USA 84, 6854-6858; Estin et al. (1988), Proc. Natl. Acad. Sci. USA 85, 1052-1056; Kantor et al. (1992), J. Natl. Cancer Inst. 84, 1084-1091; Roth et al. (1996), Proc. Natl. Acad. Sci. USA 93, 4781-4786) and/or cytokines (e.g. IL-2, IL-12), costimulatory molecules (B7-1, B7-2) (Rao et al. (1996), J. Immunol. 156, 3357-3365; Chamberlain et al. (1996), Cancer Res. 56, 2832-2836; Oertli et al. (1996), J. Gen. Virol. 77, 3121-3125; Qin and Chatterjee (1996), Human Gene Ther. 7, 1853-1860; McAneny et al. (1996), Ann. Surg. Oncol. 3, 495-500), or other therapeutic proteins.

Finally, the present invention relates to a method of producing a recombinant vaccinia virus according to the present invention, comprising the following steps: (a) generating (i) a vaccinia shuttle plasmid containing the modified F3 gene inserted at restriction site x and (ii) a dephosphorylated wt VV (VGL) DNA digested at restriction site x; (b) transfecting host cells infected with PUV-inactivated helper VV (VGL) with a mixture of constructs (i) and (ii) of step a; and (c) isolating the recombinant vaccinia viruses from the transfectants.

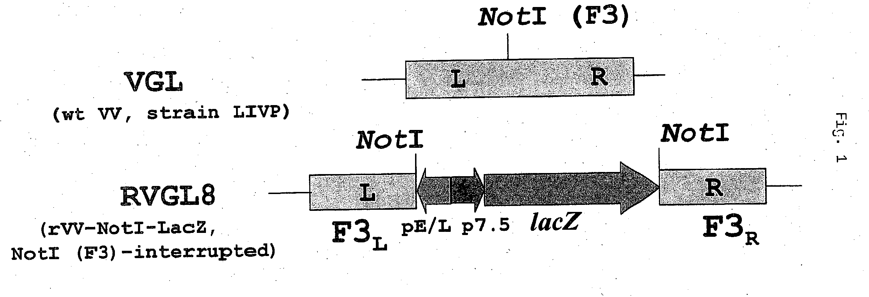

The person skilled in the art knows how to carry out said method, for example by following the instructions given in Example 1 and the legend to Figure 1; see also Timiryasova et al., Biotechniques 31 (2001), 534-540. Preferably, restriction site x is a unique restriction site. Suitable host cells are also known to the person skilled in the art and comprise many mammalian, avian and insect cells and tissues which are susceptible for vaccinia virus infection, including chicken embryo, rabbit, hamster and monkey kidney cells, e.g. HeLa cells, RK₁₃, with CV-1, Vero, BSC40 and BS-C-1 monkey kidney cells being preferred."

### Brief description of the drawings

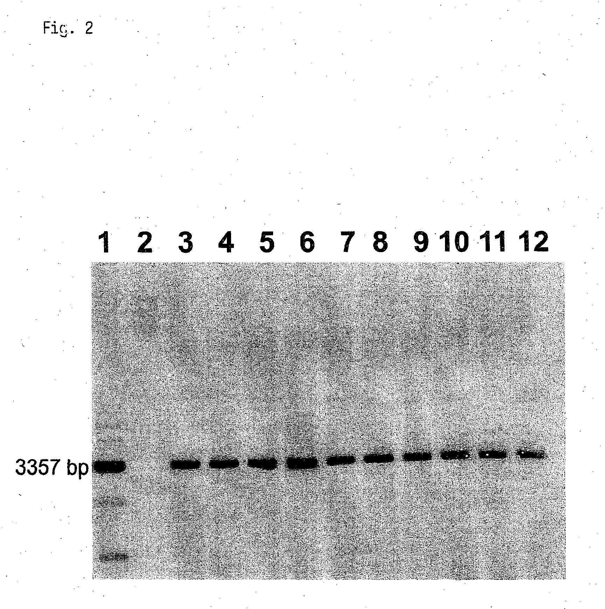

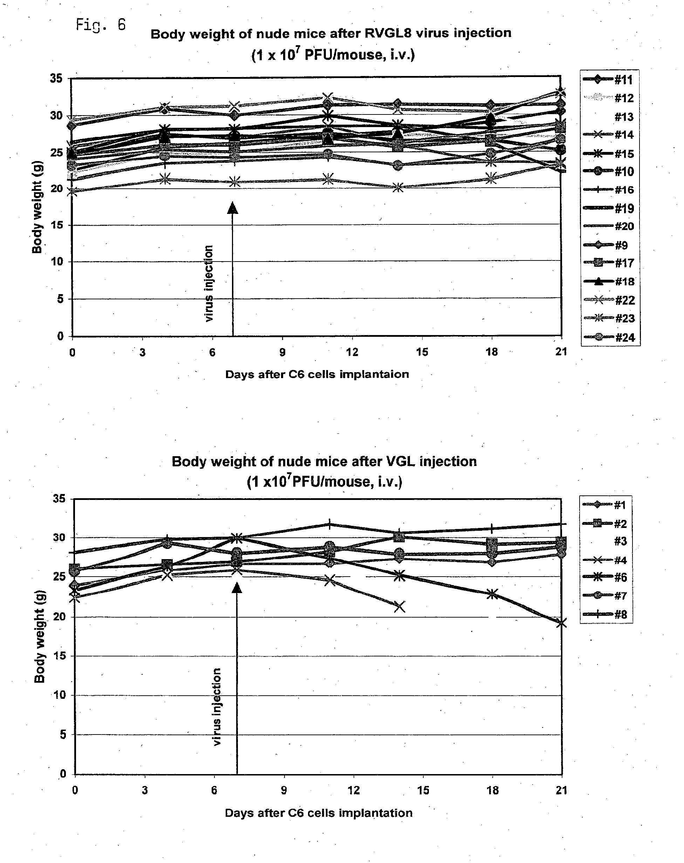

Figure 1: Schematic representation of the recombinant vaccinia virus RVGL8 used for the animal experiments
   The recombinant vaccinia virus RVGL8 was constructed by using the in vivo recombination method described in Example 1. The complex of wild-type vaccinia virus DNA digested with NotI and non-digested plasmid DNA pNZ2 was transfected for in vivo recombination into PUV-VV-infected cells. VGL, wild type vaccinia virus (strain LIVP); RVGL8, recombinant vaccinia virus encoding lacZ gene in the NotI site; Not_{L} and Not_{R}, left and right segments of unique NotI restriction site in vaccinia virus genome.; pE/L, synthetic early/late vaccinia virus promoter; p7.5, early/late vaccinia virus promoter; lacZ, lacZ gene of E. coli. Results obtained with RVGL8 are shown in Figures 2 to 8.
Figure 2: Southern blot analysis
   Total DNAs were isolated from VV and digested with NotI, electrophoresed on an 0.8% agarose gel and subjected to Southern blot analysis using a hybridization probe DNA fragment from pNZ2. The predicted size of the band is 3357 bp. Lane 1, positive control, NotI fragment (3357 bp) of plasmid pNZ2; lane 2, DNA from CV-1 cells infected with wild-type VV; lanes 3-12, DNAs from the cells infected with the single blue plaques. All DNAs of randomly selected 10 blue plaques (lanes 3-12) hybridized with the specific virus probe. As expected, the DNAs from white virus plaques (lane 2) did not hybridize with the probe. The hybridization of NotI-digested viral DNAs with a 3357 bp DNA probe confirmed the integration of the lacZ gene into *NotI* site of *virus* genome.

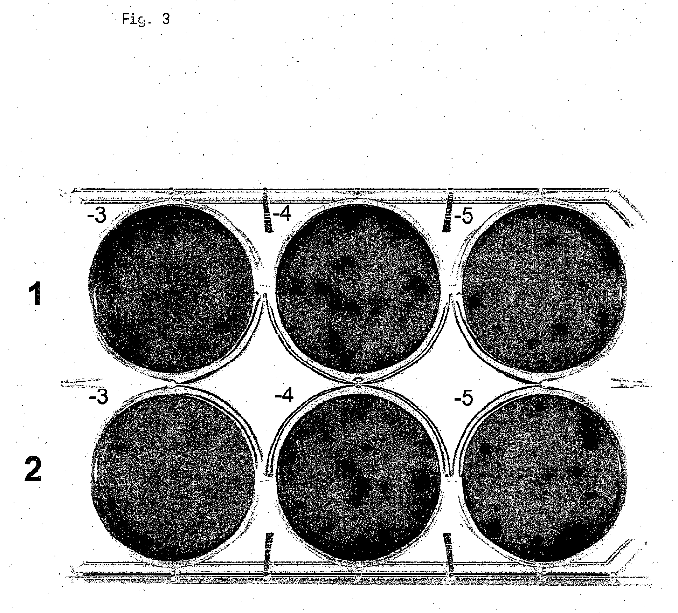

Figure 3: Expression of β-gal by vaccinia virus recombinants (RVGL8)
   CV-1 cells grown on 6-well plates were infected with tenfold dilutions of viruses obtained from a single blue plaque. After 48 h, the plates were stained with X-Gal for overnight to visualize the virus plaques obtained using the new in vivo recombination method of Example 1. 1, plaques obtained from virus stock after the transfection; 2, second purification, the single blue plaque was picked after the transfection, propagated and the cells were infected with tenfold dilutions of this virus.
Figure 4: Tumor-selective replication of vaccinia virus RVGL8
   See Example 3 for details.
Figure 5: Reduction of virulence and toxicity of vaccinia virus RVGL8
   See Example 3 for details.
Figure 6: Body weight of nude mice
   Rat glioma C6 cells at the dose of 5x10⁵/O.1 ml/mouse were implanted into nude mice on day 0. Vaccinia viruses were injected i.v. at the dose of 1x10⁷ PFU/0.1 ml/mouse on day 7. Animals were weighed twice a week.
Figure 7: Schematic representation of recombinant vaccinia viruses used for the experiments of Example 5 (Results are presented in Figures 9-12)
   TK-interrupted = RVGL7 = rVV-GFP, NotI(F3)-interrupted virus = RVGL9 =rVV-NotI-RG = rVV-RG = rW-ruc-gfp.
Figure 8: Luminescence and fluorescence images of tumor in a nude mouse
   See Example 5 for details.
Figure 9: Reduction of human breast tumor by vaccinia viruses RVGL7 and RVGL9
   See Example 5 for details.
Figure 10: Reduction of human breast tumor by vaccinia viruses RVGL7 and RVGL9
   See Example 5 for details.
Figure 11: Reduction of human breast tumor by vaccinia viruses RVGL7 and RVGL9 in nude mice
   See Example 5 for details.
Figure 12: Schematic representation of recombinant vaccinia viruses used in the experiments of Example 6
   Results obtained are shown in Figures 14 and 15; see also Table 1 for details.
Figure 13: Reduction of virulence and toxicity of several recombinant vaccinia viruses
   See Example 6 for details; recombinant vaccinia viruses used were RVGL1, RVGL5, RVGL7, RVGL8 and RVGL19
Figure 14: Reduction of virulence and toxicity of several recombinant vaccinia viruses
   See Example 6 for details

The present invention is explained by the following examples.

### Example 1: Generation of recombinant virus RVGL8

Wild type vaccinia virus strain LIVP designed as VGL was used as a parental virus for the construction of recombinant virus RVGL8. Construction of recombinant vaccinia virus RVGL8 containing lacZ gene in the NotI site was previously described in Timiryasova et al., (2001), BioTechniques 31, 534-540. Briefly, a vaccinia virus shuttle plasmid pNZ2 was created. The BamHI/SmaI fragment (3293 bp) of pSC65 (Chakrabarti et al. (1997), BioTechniques 23, 1094-1097) containing lacZ gene under the control of vaccinia p7.5 promoter and strong synthetic vaccinia pE/L promoter was isolated by digestion with restriction enzymes, blunted with Klenow enzyme, and cloned into SmaI site of pNT8 plasmid (Timiryasova et al. (2001), BioTechniques 31, 534-540), resulting in pNZ2. The pNZ2 contained the cDNA of E. coli lacZ under the control of vaccinia virus early/late promoter p7.5 and a synthetic early/late vaccinia pE/L promoter derived from pSC65 plasmid. The pNZ2 allowed for homologous recombination of lacZ into NotI site of VGL virus, resulting in the new recombinant vaccinia virus designed as RVGL8.

CV-1 cells grown on 60 mm dishes (Corning, Corning, NY, USA) were infected with PUV-VV (strain LIVP)(Tsung et al. (1996), J. Virol. 70, 165-171; Timiryasova et al. (2001), BioTechniques 31, 534-540; Timiryasova et al. (2001), J. Gene Med. 3, 468-477) at multiplicity of infection (MOI) of 1. Two hours post-infection, the cells were transfected with a mixture of NotI-digested viral DNA (4 µg) and intact plasmid DNA (4 µg). Lipid-mediated transfection of cells was carried out using 5 µl of GenePORTER reagent (Gene Therapy Systems, San Diego, CA, USA) per µg of the DNA according to manufacturers' instructions. Cells were incubated in transfection mixture for 4 h and then supplemented with a medium containing 20% of fetal bovine serum. Cytopathic effects were monitored daily by light microscopy. Cells were incubated for 5-7 days until formation of the virus plaques and complete cytopathic effect. Then, infected cells were harvested, resuspended in 0.5 ml of medium, and frozen and thawed three times to release the virus. Single virus plaques were selected for the preparation of small and large recombinant virus stocks and analyzed for the insertion and expression of the genes.

The viral DNA was analysed by Southern blots. Briefly, to isolate viral DNA, confluent monolayer of CV-1 cells, grown on 10 cm plates, were infected with the wild type VV (strain LIVP) or VV of the virus stock obtained from a single recombinant plaque. When the cytopathic effect was complete, cells were harvested and the pellet was resuspended in 3 ml of 10 mM Tris-HCl, pH 9.0. Viral particles were lysed, treated with proteinase K, and the virus DNA was isolated by phenol/chloroform extraction, followed by ethanol precipitation. The DNA was resuspended in 100 µl of sterile water. The viral DNA samples were digested by NotI overnight at 37°C, followed by phenol-chloroform treatment, precipitated and 10 µg of DNA samples were separated through a 0.8% agarose gel. The DNA was transferred to a positively charged nylon membrane (Roche Diagnostics Corporation, Indianapolis, IN, USA) and fixed to the membrane using a GS Gene Linker (Bio-Rad Laboratories, Hercules, CA, USA). The DIG-labeling of DNA was performed using a nonradioactive DNA labeling and detection kit (Roche Diagnostics Corporation) and incubating for 60 min at 37°C. The membrane was hybridized with a denatured DIG-labeled 3357 bp NotI-NotI DNA fragment of the plasmid pNZ2 encoding lacZ gene. Hybridization conditions and blot development were as suggested by the manufacturer. RVGL8 and the further recombinant vaccinia viruses used in the experiments are listed in Table 1.

**Table 1**

| **List of recombinant vaccinia viruses (VV) based on strain LIVP used in the experiments** | | | | |
|---|---|---|---|---|
| **Designation** | **Published name/names of viruses** | **Description** | **Locus of insertion** | **Reference** |
| **Designation** | **Published name(s) of viruses** | **Description** | **Locus/loci of insertion** | **Reference** |
| VGL | wt VV | strain LIVP | no insertions | |
| RVGL1 | recVV2 | (p7.5) Luc-(p11) LacZ | Hindlll-N-interrupted | Timiryasova TM, Kopylova-Sviridova TN, Fodor I. Mol. Biol. (Russian) 27:392-401 (1993); Timiryasova TM, Li J, Chen B, Chong D, Langridge WHR, Gridley DS, Fodor I. Oncol. Res. 11:133-144 (1999) |
| RVGL5 | recVV8 | (p11) LacZ | HA-interrupted | Timiryasova TM, Kopylova-Sviridova TN, Fodor I. Mol. Biol. (Russian) 27:392-401 (1993) |
| RVGL7 | rW-EGFP or rW-GFP | (PE/L) EGFP-(p7.5) LacZ | TK-interrupted | Umphress S, Timiryasova T, Arakawa T, Hilliker S, Fodor I, Langridge W. Transgenics 4:19-33 (2003) |
| RVGL8 | rW-Not-LacZ or rVV-Not-LZ | (p7.5) LacZ | Notl (F3)-interrupted | Timiryasova TM, Chen B, Fodor N, Fodor I. BioTechniques 31:534-540 (2001) |
| RVGL9 | rVV-RG or rW-ruc-gfp | (PE/L) Ruc-GFP | Notl (F3)-interrupted | Timiryasova TM, Yu YA, Shabahang S, Fodor I, Szalay AA. Proceedings of the 11^{th} International Symposium on Bioluminescence & Chemiluminescence pp.457-460 (2000) |
| RVGL19 | | (PE/L) Trf-(p7.5) LacZ in TK locus (PE/L) Ruc-GFP in F3 locus | TK- and Notl (F3)-interrupted | this invention |
| RVGL20 | | (PE/L) rTrf-(p7.5) LacZ in TK locus (PE/L) Ruc-GFP in F3 locus | TK- and Notl (F3)-interrupted | this invention |

### Example 2: Analysis of lacZ expression

Analysis of *lacZ* expression induced by recombinant RVGL8 virus was performed as described previously (Timiryasova et al. (2001), BioTechniques 31, 534-540). Briefly, CV-1 cells grown on 6-well plates (Corning, Corning, NY, USA) were infected with ten-fold dilutions of the virus stock. The virus was allowed to absorb for 1 h at 37°C with occasional rocking. Then, the virus inoculum was replaced with a complete medium containing 1% of agar, and the incubation was carried out for 48 h. To visualize the virus plaques, 300 µg of X-Gal (Molecular Probes, Eugene, Oregon, USA) per ml and 0.1% of neutral red (Sigma, St. Louis, MO, USA) were added to the second agar overlay, and plaques were counted and isolated after 12 h incubation at 37°C.

### Example 3

### Vaccinia virus recovery from tumor and organs of nude mice

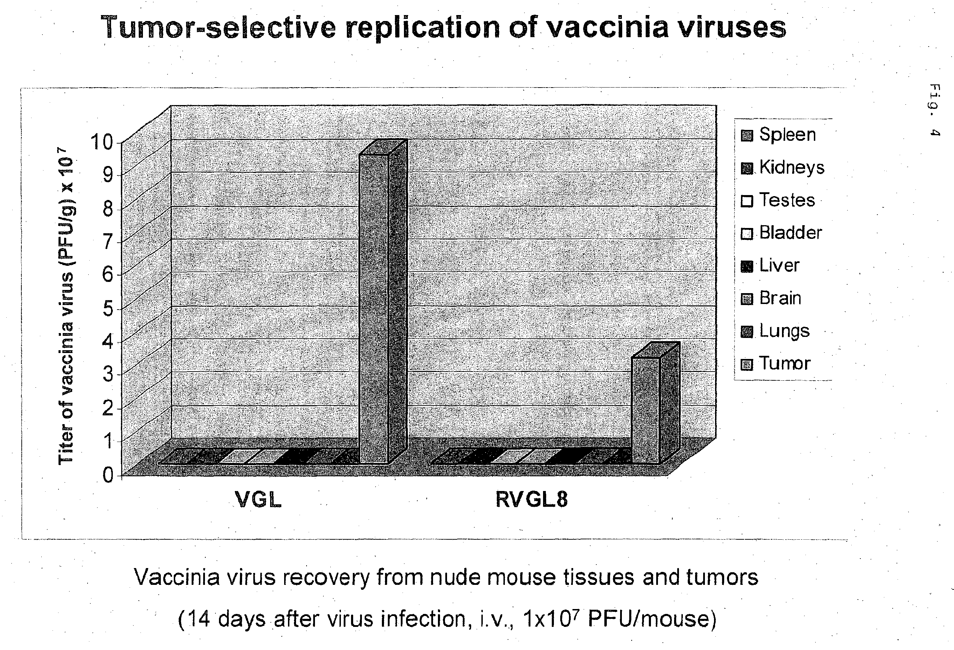

Vaccinia viruses (RVGL8; 1 x 10⁷ PFU/mouse) were intravenously injected (via femoral vein) into the mouse on day 7 after s.c. C6 cells implantation (5 x 10⁵ cells/mouse). Mice were sacrificed on day 14 after virus injection. Titer of virus was determined by plaque assay on CV-1 cells and expressed as a PFU/g of tumor or tissue. The results are shown in Figure 4.

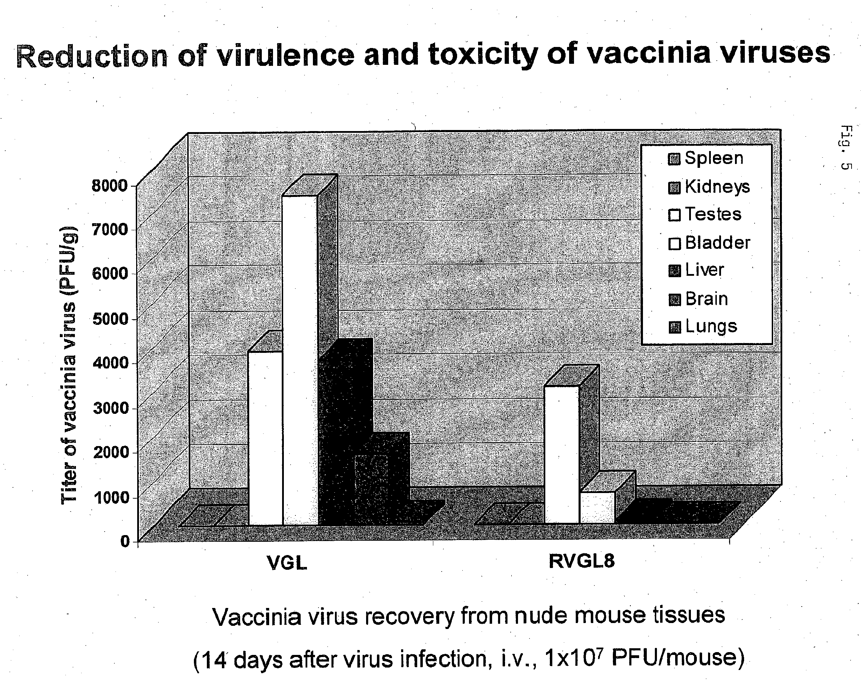

Very interesting data were received after recovering vaccinia viruses from tumor samples and different organs (see Figure 5). VGL (wt VV, strain LIVP) virus was recovered from tumor, testes, bladder, and liver and as well as from brain. However, recombinant virus RVGL8 = rVV- NotI-LacZ =NotI(F3)-interrupted was found mostly in tumor only (in mouse #24 virus was found in testes, bladder and liver; in mouse #22 in testes) and no virus was recovered from brain tissue in six tested animals. Therefore, this finding demonstrates the safety of recombinant vaccinia virus with the interruption of the NotI site located in F3 gene of vaccinia virus strain LIVP for gene therapy purposes.

### Example 4

### Reduction of human breast tumor implanted in nude mice by recombinant vaccinia viruses RVGL7 and RVGL9

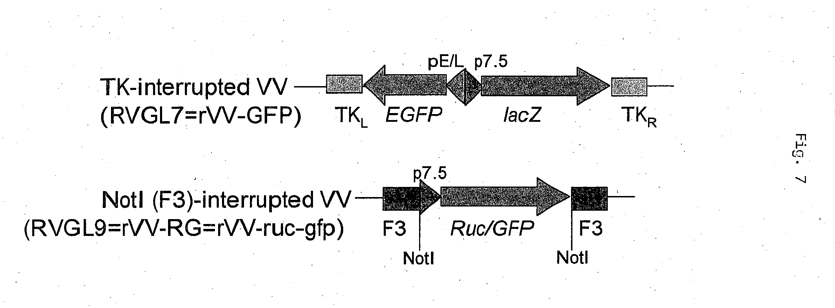

Figure 7 shows a schematic representation of the recombinant vaccinia viruses used for these experiments.

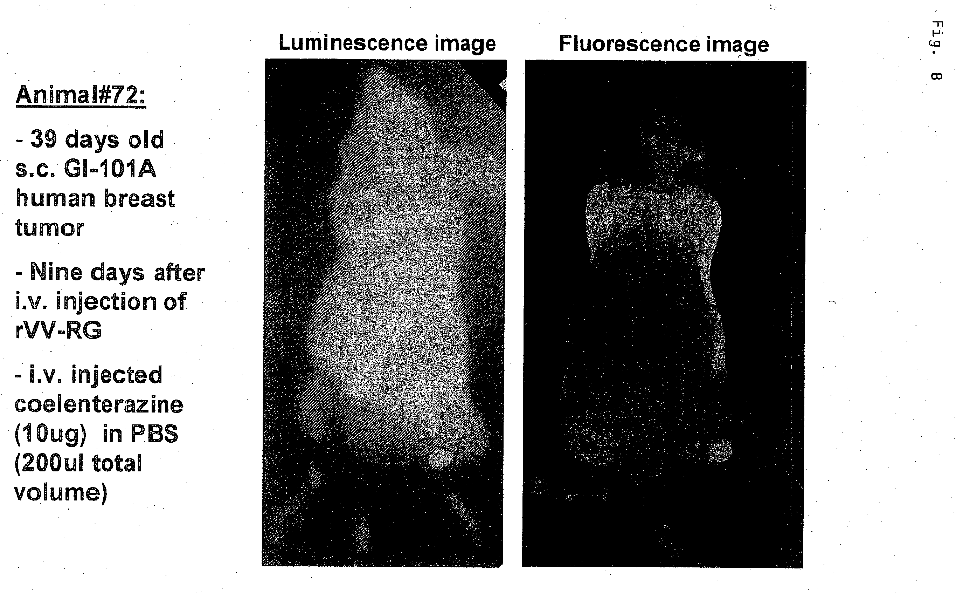

Figure 8: Luminescence and fluorescence images of tumors in a nude mouse
   Briefly, human breast GI-101A cancer cells (5x10⁶ cells/mouse) were subcutaneously implanted into the right thigh of the mice. Thirty days after cell implantation, NotI (F3)-interrupted virus expressing fusion of *Renilla* luciferase and green fluorescence protein (RVGL9 = rW-RG = rWruc-gfp) was injected intravenously via tail vein at the dose of 1x10⁷ PFU/mouse. Fluorescence image of GFP and low-light image of luciferase expression was taken nine days after virus injection, i.e. 39 days post cell implantation. The results are shown in Figure 8.

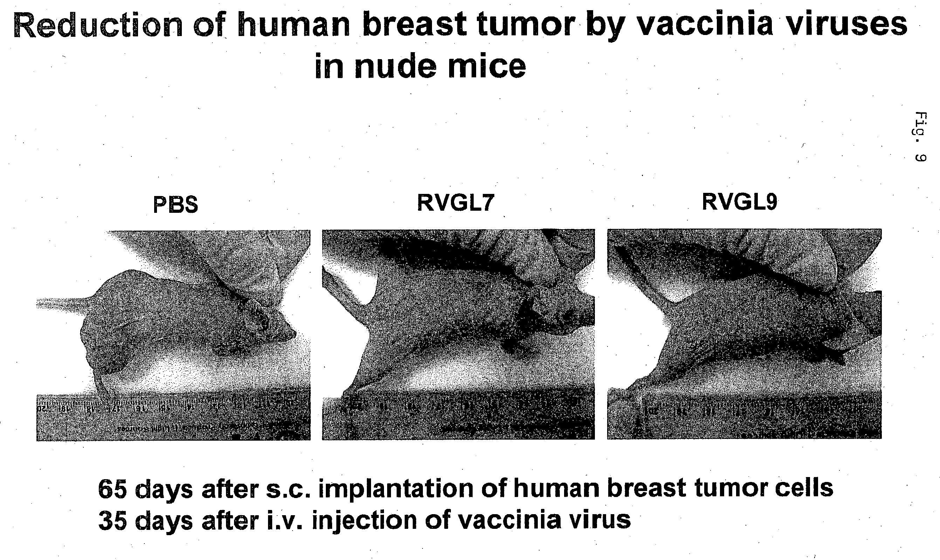

Figure 9: Reduction of human breast tumor implanted into nude mice by vaccinia viruses RVGL7 or RVGL9
   Human breast GI-101A cancer cells (5 x 10⁶ cells/mouse) were subcutaneously implanted into right thigh of the mice. Mice were injected i.v. with RVGL7=rVV-GFT=TK- or RVGL9=rVV-ruc-gfp=NotI (F3)-interrupted viruses (1 x 10⁷ PFU/mouse in 0.1 ml) and PBS control on day 30 after cell implantation. Images were taken on day 65 after GI-101A cell implantation and 35 days after virus or PBS injection. The results presented in this figure demonstrate drastic reduction of tumor volume in the mice injected with TK- or NotI (F3)-interrupted vaccinia viruses to compare with the tumor in the mice injected with PBS. One representative of each group is shown.

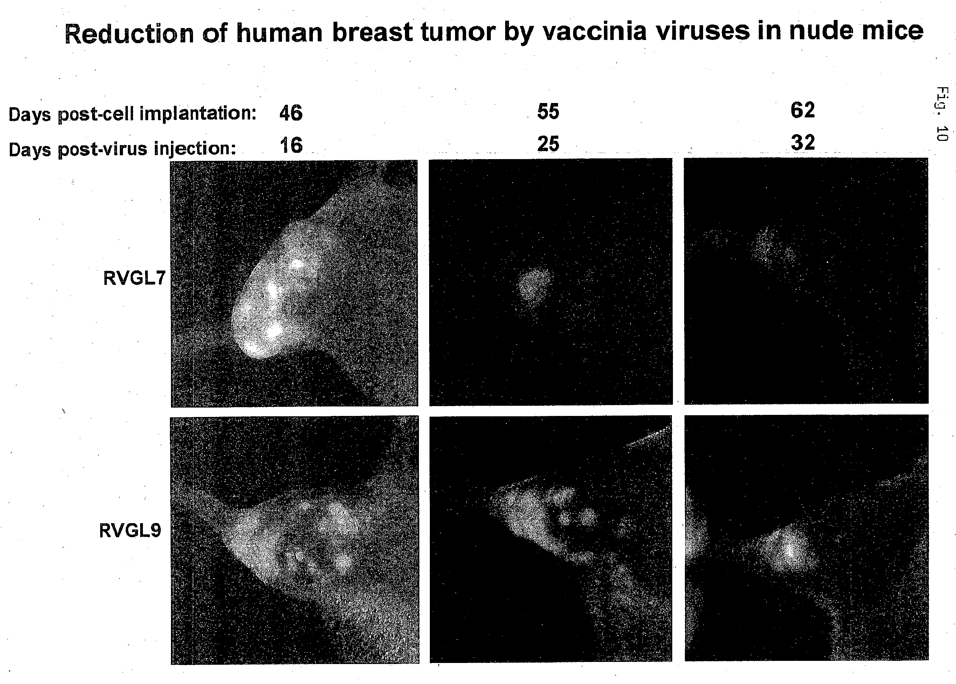

Figure 10: Human breast GI-101A cancer cells (5 x 10⁶ cells/mouse) were subcutaneously implanted into right thigh of the mice. Mice were injected i.v. with RVGL7=rW-GFP=TK- or RVGL9=rVV-RG=rVV-ruc-gfp=NotI (F3)-interrupted viruses (1 x 10⁷ PFU/mouse in 0.1 ml) on day 30 after cell implantation. The data demonstrate GFP expression in tumor area in the mice injected wich TK⁻ or NotI (F3)-interrupted vaccinia viruses. No GFP signals were observed in other parts of mice body. Expression of GFP can be visualized as early as 48 h after virus injection through tail vein. On day 16 after virus injection very strong signals of GFP were observed, which correspond to a tumor volume of about 1300-1620 mm³ for TK- or NotI(F3)-interrupted virus, respectively, and reduced GFP signals on day 25 (1218-1277 mm³ for TK- or NotI(F3)-interrupted virus, respectively) and 32 (514-887 mm³ for TK- or NotI(F3)-interrupted virus, respectively) due to reduction of tumor volume.

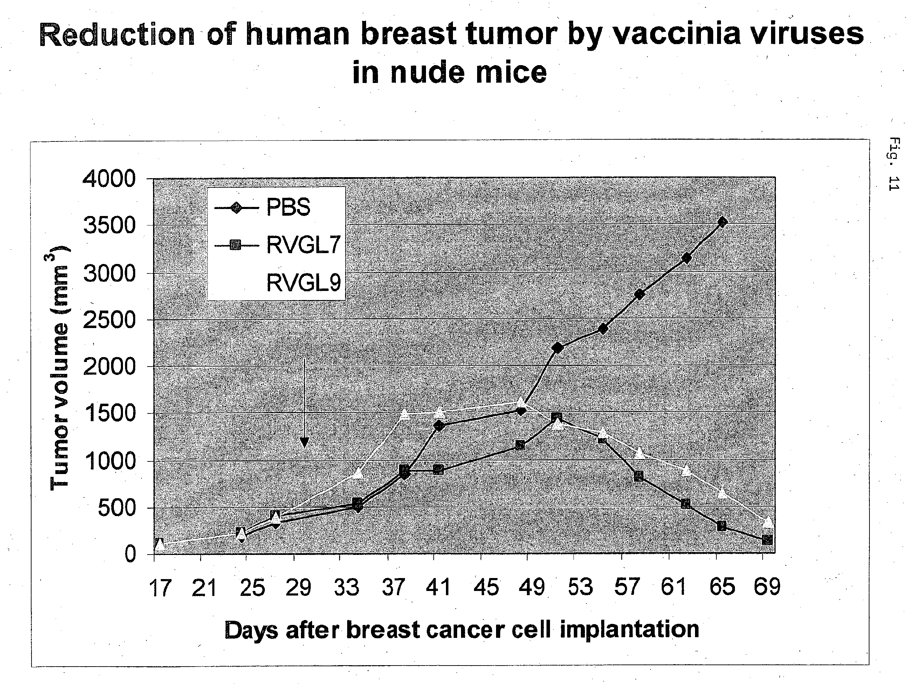

Figure 11 GI-101A breast cancer cells were implanted subcutaneously into the right thigh of 4-5-week old female athymic (nu/nu) mice in the dose of 5x10⁶ cells/mouse. Thirty days after tumor implantation, when the tumor reached about 500 mm³ in volume, a single dose (1x10⁷ PFU/mouse in 0.1 ml) of RVGL7 = rVV-GFP = TK- or RVGL9 = rW-RG = rVV-ruc-gfp = NotI (F3)-interrupted vaccinia viruses or PBS control was injected intravenously (via tail vein). Tumor dimensions were measured with vernier caliper twice a week and volumes were calculated as (LxHxW)/2, where L, H and W represent the length, width, and height of the tumor, respectively and expressed in mm³. The data from this experiment clearly demonstrate significant (60-80% on day 65) tumor reduction in the mice injected with TK-, NotI(F3)-interrupted vaccinia viruses. In contrast, tumors grew very rapidly in the mice injected with PBS. Arrow shows the day of PBS or TK-, NotI(F3)- interrupted viruses injection.

### Example 5

### Reduction of vaccinia virus toxicity and virulence

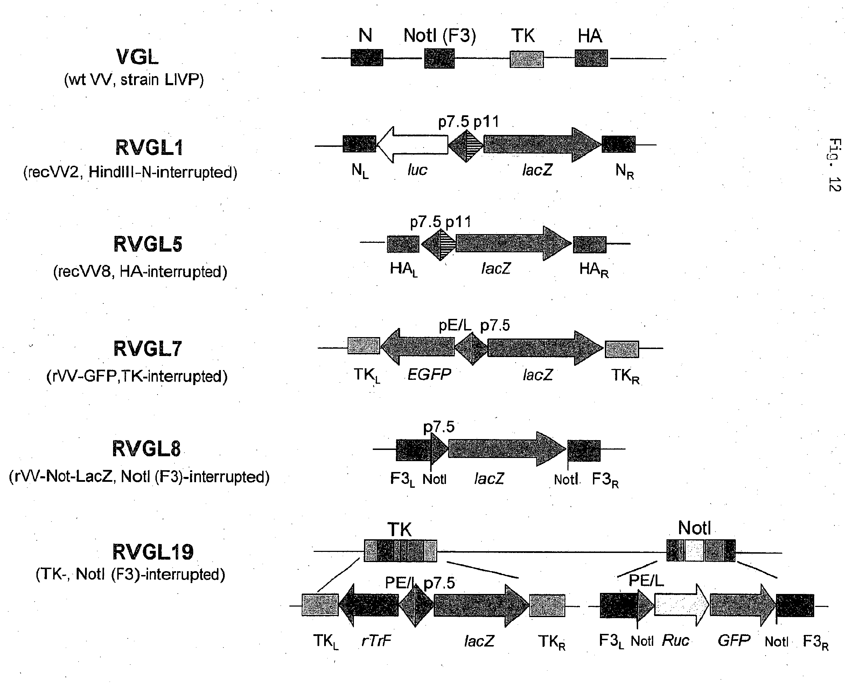

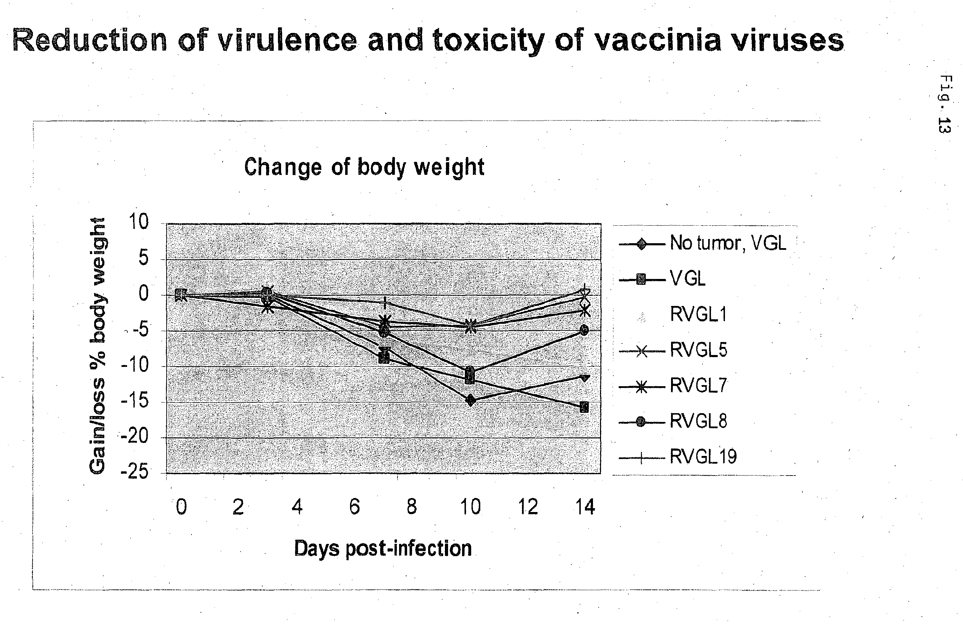

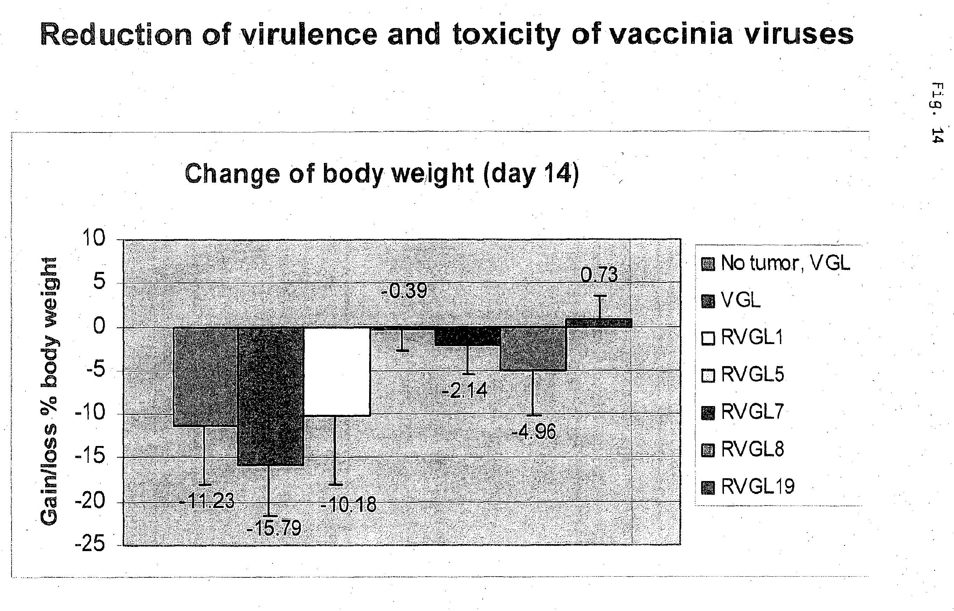

Figure 12 shows a schematic representation of the recombinant vaccinia viruses used for these experiments. The results are shown in Figures 13 and 14.
Figure 13: Rat glioma C6 cells at the dose of 5x10⁵/0.1 ml/mouse were implanted s.c. into the right thigh of nude mice (5-6 old male mice) on day 0. Vaccinia viruses were injected i.v. (via tail vein) at the dose of 1 x 10⁷ PFU/0.1 ml/mouse on day 7. Animals were weighed twice a week. Injection of VGL (wild type vaccinia virus, strain LIVP), RVGL5 (HindIII-N-interrupted) causes toxicity in nude mice: mice continue to lose the weight. However, recombinant vaccinia viruses RVGL5 (HA-interrupted), RVGL7 (TK-interrupted), RVGL8 ( NotI(F3)-interrupted), RVGL19 (double, TK- and NotI(F3)-interrupted) were less toxic in nude mice: after losing some body weight, 10 days post-infection, mice started to gain the body weight.
Figure 14: Rat glioma C6 cells at the dose of 5x10⁵/0.1 ml/mouse were implanted s.c. into the right thigh of nude mice (5-6 old male mice) on day 0. Vaccinia viruses were injected i.v. (via tail vein) at the dose of 1 x 10⁷ PFU/0.1 ml/mouse on day 7. Gain/loss of body weight on day 14 post infection was calculated as the percentage: body weight - tumor weight on day of virus injection (g) / body weight-tumor weight on day 14 (g) x 100%.

Injection of VGL virus (wild type vaccinia virus, strain LIVP) or RVGL1 virus (HindIII-N-interrupted) causes toxicity in nude mice. Mice without tumor injected with VGL (wt VV, strain LIVP) lost 11.23% of body weight. Mice bearing tumor injected with VGL (wt VV) or with RVGL1 (HindIII-N-interrupted) lost 15.79% and 10.18% of body weight, respectively. Tumor-bearing mice injected with RVGL5 (HA-interrupted), RVGL7 (TK-interrupted), RVGL8 (NotI(F3)-interrupted) on day 14 after virus injection lost only 0.39, 2.14, and 4.96% of body weight, respectively. However, tumor-bearing mice injected with virus carrying double gene interruption, RVGL19 (double, TK- and NotI(F3)-interrupted) demonstrated 0.73% gain of body weight compared to the body weight on day 0. Therefore, based on the results of body weight, it can be concluded that single interruption of HA, TK, F3 (NotI site) and double interruption of TK and F3 (NotI site) genes in vaccinia virus genome reduces virulence and toxicity of the vaccinia virus strain LIVP.

## Claims

1. A recombinant vaccinia virus wherein (a) the F3 gene and (b) the TK gene and/or HA gene have been modified.

2. The recombinant vaccinia virus of claim 1, wherein (a) the F3 gene and (b) the TK gene and/or HA gene have been inactivated.

3. The recombinant vaccinia virus of claim 1 or 2, wherein at least the F3 gene has been inactivated by insertion of a foreign DNA sequence.

4. The recombinant vaccinia virus of claim 3, wherein the F3 gene and the TK gene have been inactivated by insertion of a foreign DNA sequence.

5. The recombinant vaccinia virus of any one of claims 1 to 4, wherein said modification of the F3 gene is at the NotI site within the F3 gene at position 35 or at position 1475 inside of HindIII-F fragment of vaccinia virus DNA strain LIVP.

6. The recombinant vaccinia virus of any one of claims 1 to 5 which has been derived from VV strain LIVP.

7. The recombinant vaccinia virus of any one of claims 3 to 6, wherein the foreign DNA sequence encodes a protein to be expressed under the control of a regulatory sequence.

8. The recombinant vaccinia virus of claim 7, wherein the regulatory sequence comprises the vaccinia virus early/late promoter p7.5.

9. The recombinant vaccinia virus of claim 7 or 8, wherein the regulatory sequence (additionally) comprises a synthetic early/late vaccinia pE/L promoter.

10. The recombinant vaccinia virus of any one of claims 3 to 9, wherein the foreign DNA sequence encodes a detectable protein or a protein capable of inducing a detectable signal.

11. A host cell containing a recombinant vaccinia virus according to any one of claims 1 to 10.

12. A pharmaceutical composition containing a recombinant vaccinia virus of any one of claims 1 to 10 and a suitable carrier.

13. Use of (a) a recombinant vaccinia virus according to any one of claims 1 to 10 or (b) a recombinant vaccinia virus having a modified F3 gene, TK gene or HA gene, for the preparation of a pharmaceutical composition for gene therapy or vaccine therapy.

14. Use according to claim 13, wherein the F3 gene, TK gene and/or HA gene has been inactivated.

15. Use according to claim 14, wherein the F3 gene, TK gene and/or HA gene has been inactivated by insertion of a foreign DNA sequence.

16. Use according to any one of claims 13 to 15, wherein said gene therapy is cancer gene therapy.

17. A method of producing a recombinant vaccinia virus according to any one of claims 3 to 10, comprising the following steps:
(a) generating (i) a vaccinia shuttle plasmid containing the modified F3 gene inserted at restriction site x and (ii) a dephosphorylated wt VV (VGL) DNA digested at restriction site x;
(b) transfecting host cells infected with PUV-inactivated helper VV (VGL) with a mixture of constructs (i) and (ii) of step a; and
(c) isolating the recombinant vaccinia viruses from the transfectants.

18. The method of claim 17, wherein the host cells are CV-1 cells.
